# EUROPEAN PATENT APPLICATION

(11) **EP 1 964 923 A1**
(43) Date of publication of application: **03.09.2008**
(21) Application number: 07003942.5
(22) Date of filing: 27.02.2007
(51) Int. Cl.: C12P 1/00, C12P 7/00, C12P 21/00

(54) **Multiplex fermentation process**

(71) Applicant: Vertès, Alain André Guy, 4052 Basel (CH)
(72) Inventor: Vertès, Alain André Guy, 4052 Basel (CH)

(57) **Abstract**

The present invention provides a process of fermenting suitable carbon sources to produce more than one useful substance (i) in parallel and/or (ii) in sequence using various cellular compartments such as, and where applicable, (a) extra-cellular compartment (b) intracellular compartment (c) cellular membrane (d) periplasm (e) specialized organelle e.g, mitochodria or chloroplasts. Preferred embodiments of the invention include (i) growing microbial cells and co-producing in parallel more than one useful substance (ii) growing microbial cells and producing in sequence more than one useful substance (iii) growing microbial cells producing one or more useful substance and subsequently collecting said cells, incubating them under non-replicating conditions and producing one or more useful substance. The invention is particularly useful to capture economies of scope via producing by microbial fermentation commodity chemicals, such as ethanol or chemical building blocks, and high-value fine chemicals such as amino acids, vitamins, proteins, enzymes, or pharmaceuticals. The invention also relates to processes of producing fermentation products including the fermentation process of the invention.

## Description

### FIELD OF THE INVENTION

The present invention relates to fermentation processes for producing fermentation products including, but not limited to, pharmaceuticals, food supplements, feed supplements, proteins, peptides, vitamins, amino acids, organic acids, ethanol, butanol, acetone.

### BACKGROUND OF THE INVENTION

Fermentation processes are used for producing a vast array of useful substances, that are either commercial products (e.g., pharmaceuticals, proteins including pharmaceutical biologics or industrial enzymes, peptides, vitamins including beta-carotene, riboflavin, or vitamin B12), amino acids (e.g., glutamic acid, lysine, tryptophane), biochemical buildings blocks (e.g., 1,4 diacids: succinic acid, fumaric acid, and acetic acid, organic acids, 2,5-furan dicarboxylic acid, 3-hydroxy propionic acid, aspartic acid, glucaric acid, glutamic acid, itaconic acid, levulinic acid, 3-hydroxyburyrolactone, glycerol, sorbitol, xylitol, arabinitol, lactic acid, citric acid, gluconic acid), or commodity chemicals (e.g., ethanol, butanol, acetone).

There is a need for improved fermentation processes as well as for processes that include a fermentation step, as exemplified by the observation that production of commodity chemicals by fermentation using renewable materials competes on a cost-basis with chemical or petrochemical processes. As such, there is a need for improved fermentation processes that enable improved process economics. Moreover, production by fermentation enables the production of biochemical compounds with the same chiral properties, rather than constituting racemic mixtures.

### SUMMARY OF THE INVENTION

The present invention provides fermentation processes that are improved for producing various useful substances from materials providing a source of carbon such as sugar-, starch-, cellulose-, or hemicellulose-containing materials. Typically, said source of carbon can be pre-treated or simultaneously treated with various enzymes such as to make the sugars directly metabolizable by the fermentation organism used. Typical fermentation processes of the invention produce more than one desired product in parallel or in sequence (i.e., using the same cellular culture utilized either in a batch, fed-batch, semi-fed-batch, semi-continuous or continuous process, or a combination thereof), using either one or more of the various cellular compartments available, including but not limited to: intra-cellular compartment, extra-cellular compartment, cellular membrane, periplasm (for example in the case of Gram-negative bacteria such as *Escherichia coli,* where particularly proteins accumulate as inclusion bodies), or specialized organelles (such as, depending on the organism considered, mitochondria or chloroplasts).

The invention relates to a process where a suitable source of carbon as described above is subjected to a fermenting microorganism.
In a first embodiment, said microorganism is grown and co-produces in parallel more than one useful substance.
In a second embodiment, said microorganism is grown and produces in sequence more than one useful substance.
In a third embodiment, said microorganism is grown while producing a useful substance as described above, cells are subsequently collected and incubated in non-replicating conditions during which phase they are utilized to produce one or more different useful substances. Fermentation products are recovered by appropriate downstream processing techniques known to persons skilled in the art.
For example, in the first embodiment, cells and extracellular phase can be separated by centrifugation. Cells containing in one or more compartment one of more useful substances are subsequently broken open, for example by means of a French press device, and the useful substances recovered. A particular example is the recovery of proteins from the periplasm of *E. coli* in the form of inclusion bodies. In addition, the product secreted in the extracellular phase is recovered by appropriate techniques, for example by distillation. In a preferred embodiment, the useful substance produced in the extracellular compartment is a commodity chemical such as ethanol, butanol, or acetone, or a chemical building block such as 1,4 diacids: succinic acid, fumaric acid, and acetic acid, organic acids, 2,5-furan dicarboxylic acid, 3-hydroxy propionic acid, aspartic acid, glucaric acid, glutamic acid, itaconic acid, levulinic acid, 3-hydroxyburyrolactone, glycerol, sorbitol, xylitol, arabinitol, lactic acid, citric acid, gluconic acid).
In the second embodiment, cells produce useful substances that are secreted in the first fermentation medium. Said first fermentation medium and cells are separated at the end of the initial production phase. Useful substances are recovered from the spent fermentation medium by downstream processing techniques known to people skilled in the art. Said cells are re-suspended in a second fermentation medium and subjected to an external stimulus to initiate the second fermentation. Such stimuli include various physicochemical stimuli including, but not limited to: a different incubation temperature, additions of one or more specific compounds, variation in cell catalyst concentration -including particularly using very high cell concentrations that are for example, 5 to 50 times higher than the cell concentration reached at the end of the first production phase. In a preferred embodiment, useful substances first produced are an amino acid, a vitamin or a peptide, useful substance secondly produced is ethanol or organic acid, and fermenting organism is a recombinant *Corynebacterium* or a recombinant *Bacillus,* such as *Corynebacterium glutamicum* or *Bacillus subtilis.*
In the preferred third embodiment, cells are re-suspended in a second fermentation medium under conditions that repress cell replication and subjected to an external stimulus as described in the second embodiment. Useful substances first produced are an amino acid, a peptide, or a vitamin, and useful substances produced secondly is ethanol, and fermenting organism is a recombinant *Corynebacterium* or a recombinant *Bacillus,* such as *Corynebacterium glutamicum* or *Bacillus subtilis.*

### BRIEF DESCRIPTION OF THE DRAWINGS

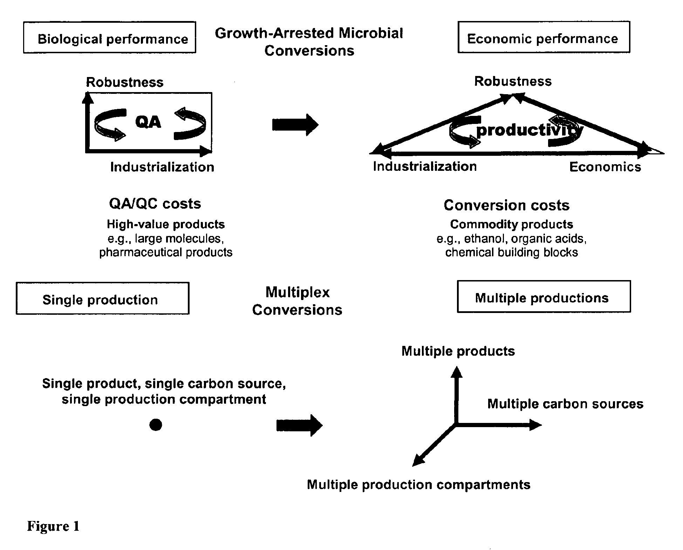

**Figure 1****: Multiplex fermentation concept.** The multiplex fermentation concept refers to a process where more than one fermentations are performed during the same manufacturing operations. For example, proteins or amino acids could be produced during the first stage of a two-stage process where cells would be collected and reused for commodity production such as lactate, succinate, or ethanol. Notably, *E. coli* offers the possibility to accumulate proteins in its periplasm as inclusion bodies that could be recovered following a commodity production phase. The key implementation challenge is to develop suitable downstream purification procedures and genetic signals to turn on and off at the adequate time the desired productions. In addition, the metabolism of the fermentor or the combination of products is optimized to ensure satisfactory yields via achieving suitable intracellular energy levels and reducing equivalent balances. Energy levels and reducing equivalent balances can be set at the appropriate level by metabolic engineering techniques or by adapting the composition of the fermentation medium. The multiplex fermentation format operates a dramatic change in throughput by performing multiple manufacturing operations in parallel or in sequence using the same cell culture, thereby capturing economies of scope. Moreover, this format is intrinsically flexible and thus facilitates manufacturing operations management, for instance by enabling production campaign schedules to closely adapt to market demands or logistics constraints. In addition, this inherent flexibility represents a key success factor regarding capital expenses as compared to the petrochemical industry, for biotechnological productions can be switched by a simple change of fermentor strain or environmental stimuli. A survey of the existing literature demonstrates that biotechnological processes developed to this date do not integrate multiple productions of useful substances

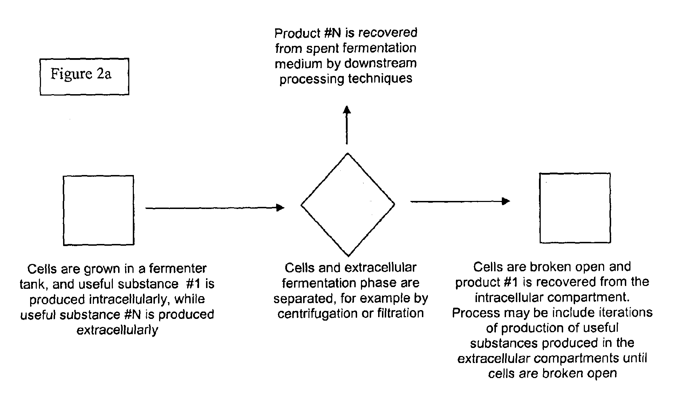

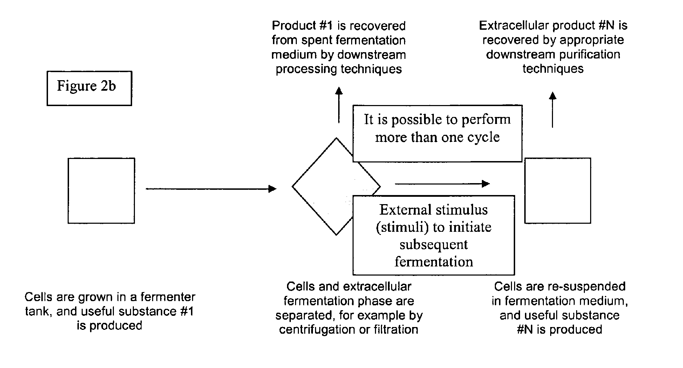

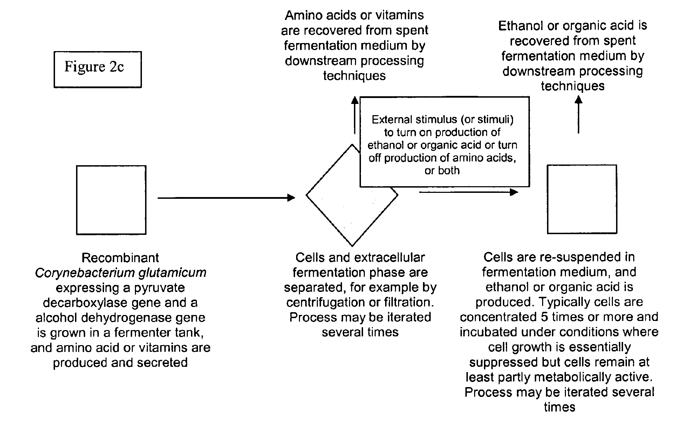

**Figure 2****: Typical embodiments of the multiplex fermentation invention.**

### DETAILED DESCRIPTION OF THE INVENTION

The present invention provides improved fermentation processes which are suitable for producing more than one useful substance using the same cellular culture. Particularly, the present invention provides novel fermentation processes for the production of ethanol, for example for use as fuel, in combination with the production of one or more additional useful substance. Appropriate genetic signals or external stimuli are used to turn on and off the various production phases at the appropriate times. In addition, the metabolism of the fermentor organism or the combination of products is optimized by metabolic or by genetic engineering or a combination thereof to ensure satisfactory yields via suitable intracellular energy levels and reducing equivalent balances.

### General background

Biofuels, including biodiesel (methyl/ethyl esters), ethanol, and hydrogen constitute attractive substitutes of petroleum-derived fuels for transportation. Applied microbiology provides practical solutions to manufacture fuels from renewable biomass at the industrial scale. For example, the worldwide production of biotechnological ethanol has reached 150 million hectolitres in 2004 using the yeast *Saccharomyces cerevisiae* to ferment maize starch-derived glucose or saccharose extracted from sugar cane (Soetaert & Vandamme, 2006; Karlscheuer et al., 2006). In addition to starch and saccharose, various sources of carbohydrates can be used for fermentation, including inulin, lignocellulose and carbohydrate-containing residues such as whey or sulphite spent liquor (Peters et al., 2006). Lignocellulosic biomass is cheap and abundant and therefore represents a very attractive primary raw material. Particularly, biomass sourced from switch-grass, short rotation woody plants, or municipal solid wastes are ideal candidate raw materials. Noteworthily, economies of scale, economies of scope and economies of skills can be derived from densely integrated bioconversion processes. Biorefineries represent such integrated structures with the capacity to manufacture an array of compounds and to serve different industries such as the chemical, food, feed, cosmetic, or pharmaceutical industry (Clements et al., 2006; Thomsen et al., 2005, Webb et al., 2004).

### Current ethanol production technology and lignocellulosic ethanol biorefinery

Nowadays, virtually all biotechnological ethanol fuel manufactured at the industrial scale is produced through fermentation of relatively easily accessible sugars by the yeast *S. cerevisiae.* Baker's yeast advantages for industrial ethanol production include its long history of safe use, a native high ethanol tolerance (up to 15% ethanol), and high final ethanol concentration (10-12%). Moreover, its sugar conversion rates are close to the theoretical maximum (95%) and its low pH tolerance helps prevent the growth of spoilage organisms. On the other hand, the robustness of yeast-based processes is limited by the sensitivity of *S. cerevisiae* to temperatures higher than 35°C, to the contamination of lactic acid bacteria, and to glucose repression phenomena that regulate its central metabolism. Moreover, the growth of *S. cerevisiae* is inhibited by a variety of compounds generated during the saccharification of lignocellulosic material and it cannot ferment xylose or arabinose. These two pentoses represent a significant portion, respectively 20% and 5% (Aristidou et al., 2000), of the sugars comprised in lignocellulosic biomass. Notably, actively growing yeasts produce ethanol up to 33 times faster than stationary cells (Bellissimi et al., 2005).

### Multiplex Fermentation

In the Multiplex Fermentation design, subject of the present invention, high-value products (e.g., proteins in soluble or inclusion body form, or fine chemicals such as amino acids) are produced using pure cultures or multi-cultures and multi-step fermentations. This parallel utilization for production of more than one cellular compartment has not been attempted to date and thus represents an attractive lead to design densely integrated bioconversions. For example, the cellular membrane, the extracellular space and the intracellular space could be used to produce high value products, while the extracellular space is used for the production of the commodity products of interest, as it is from this production compartment that downstream processing is the most straightforward. Likewise, uncoupling of the cell catalyst production phase and of the product production phase can be envisaged, as a means not only to alleviate the impact of growth inhibitors that result from raw materials pre-treatment steps (Klinket et al, 2004), but also to improve total yield and raw material yield (Vertès et al., 2005).

In addition to native ethanol producers such as *S. cerevisiae* or *Zymomonas mobilis* (Doelle et al., 1993), several organisms capable of degrading most of the sugars present in lignocellulosic biomass have been engineered for ethanol production, and particularly *Escherichia coli* (de Carvalho et al., 2002; Ingram et al., 1999) or *Klebsiella oxytoca* (Dien et al., 2003). However, despite *E. coli* exhibits the advantage of being able to grow at temperatures up to 42°C using a large range of carbohydrates, it is less hardy that *S. cerevisiae,* having a narrow and neutral pH range and relatively low ethanol tolerance. Notably, these conditions are permissive to the growth of numerous spoilage microorganisms. While antibiotics could be used to minimize spoilage, this approach does not represent an economically viable option for the manufacture of commodity chemicals with low bulk values, For such chemicals, systems that are less permissive to growth of spoilage organisms are necessary - for example using growth-arrested cells as discussed. Nevertheless, adequate advances in ethanol separation ethanol separation technology (e.g., combined distillation with adsorption (Gulati et al., 1996), pervaporation (O'Brien et al., 2004), or stripping (Taylor et al., 2000) could maintain ethanol recovery steps cost-effective even from fermentation streams at 5% final ethanol concentrations. This makes possible the use of organisms with lower ethanol resistance than *S. cerevisiae,* but with biotechnological attributes that more closely match the requirements of densely integrated biorefineries. An alternative strategy consists in selecting industrial ethanol production organisms not based on their native ethanol secretion properties or sugar utilization ranges, but rather on a set of intrinsic industrial properties, such as outstanding robustness, rapid growth rate, hardiness, high industrialisation properties and public acceptance. Food organisms such as corynebacteria or lactic acid bacteria typically exhibit all of these attributes (Inui et al., 2004; Talarico et al., 2005; Nichols et al., 2003; Bothast et al., 1999), and thus typically naturally address issues that reduce yeast-based processes profitability (limited sugar utilization range, requirement for traces of molecular oxygen, coupling between cellular growth and ethanol production, sensitivity to growth-inhibitors generated at the pre-treatment step of lignocellulosic biomass, sensitivity to contamination and limited temperature range).

Furthermore, the industrial potential of continuous processes or of processes with cell reuse has largely been ignored, as to this date batch fermentation remains the preferred process for ethanol production. Notably, higher volumetric productivities in excess of 40g/l/h can be achieved in a continuous fermenter (Nigam et al., 2000), which combines to other industrial advantages of continuous fermentations including decreased capital needs and decreased production inputs (manpower, reduced downtime, and reduced utility costs) Keim et al., 1983). Another technological option is to use growth-arrested cell catalysts (Fig. 2) incubated at very high cellular densities for several production cycles in either batch or continuous mode. Under these incubation conditions, the cellular replication of anaerotolerant or quorum sensing sensitive organisms is essentially arrested (Inui et al., 2004; Inui et al., 2004b; Vertès et al., 2005). As exemplified using *Corynebacterium glutamicum* (Inui et al., 2004; Inui et al., 2004b; Vertès et al., 2005), this design which uncouples cell growth and product production offers the benefit to optimize raw material yield, as energetic expenses for vegetative functions are minimized. Notably, hypoxic conditions are very simple to achieve under manufacturing conditions, as it is homogenous oxygenation which is oftentimes costly to maintain. The linear relationship that exists between cell density and yield, and the intrinsic simplicity of the design enables its application to numerous product production by various microbial genera through processes characterized by fast on/off responses, reduced sensitivity to the presence of growth-inhibitors, and reduced heat generation rates (Vertès et al., 2005). Alternative mechanisms can also be used to arrest cellular replication, such as controlled expression of essential cellular division genes, as observed using an *ftsZ Bacillus subtilis* mutant (Park et al., 2005). Notably, *S. cerevisiae* is ill-suited for use in such schemes as it requires traces of molecular oxygen (Rosenfeld et al., 2003). This latter observation reinforces the view that alternative ethanol producing bioconverters represent critical technological options.

### Preferred embodiments

### Figure 2 shows preferred embodiments of the invention.

In the first embodiment (Figure 2a), cells and extracellular compartment can be separated by centrifugation or filtration. Cells containing in one or more compartment one of more useful substances may be reused to produce an additional useful substance or are subsequently broken open, for example by means of a French press device, and the useful substances recovered from the intracellular compartment. A particular example is the recovery of proteins from the periplasm of *E. coli* in the form of inclusion bodies. In addition, the product secreted in the extracellular phase is recovered by appropriate techniques, for example by distillation. In a preferred embodiment, the useful substance produced in the extracellular compartment is a commodity chemical such as ethanol, butanol, acetone, or an organic acid.

In the second embodiment (Figure 2b), cells produce useful substances that are secreted in the first fermentation medium. Said first fermentation medium and cells are separated at the end of the initial production phase. Useful substances are recovered by downstream processing techniques known to people skilled in the art. Said cells are re-suspended in a second fermentation medium and subjected to at least one external stimulus to initiate the second fermentation. Such stimuli include various physicochemical stimuli including, but not limited to: a different incubation temperature, additions of one or more specific compounds, variation in cell concentration -including particularly using very high cellular concentration that are for example, 5 to 50 times higher than the cellular concentration reached at the end of the first production phase, changes in dissolved oxygen tension, removal or sequestration of a growth stimulator, etc. In a preferred embodiment, useful substances first produced are an amino acid, a vitamin or a peptide, useful substance secondly produced is ethanol or an organic acid, and fermenting organism is a recombinant *Corynebacterium,* a recombinant *Lactobacillus,* or a recombinant *Bacillus,* such as *Corynebacterium glutamicum, Lactobacillus lactis* or *Bacillus subtilis.*

In the preferred third embodiment (Figure 2c), useful substances first produced are an amino acid, a peptide, or a vitamin, and useful substance produced second is ethanol, and fermenting organism is a recombinant *Corynebacterium* or a recombinant *Bacillus,* or *Lactobacillus,* such as *Corynebacterium glutamicum* or *Bacillus subtilis,* or *Lactobacillus lactis.* Cells are incubated for production of the last useful substance production under conditions on repressed cell division, achieved using a bacteriostatic condition, such as addition of a bacteriostatic agent, changes in oxygen tension, or re-suspension of cells to reach high cell densities, typically 5 to 50 times higher that those attained routinely at the end of the logarithmic phase of growth in media and under incubation conditions typically used to grow the type strain of the organism considered.

### Fermentation

Fermentation refers to any process comprising a fermentation step. A fermentation process of the invention refers to processes for the production of two of more useful substances. Fermentations are typically conducted in tanks or fermenters, and may be conducted either in a batch or fed-batch format, or in a continuous format or semi-continuous format, with or without concomitant growth, or any combination thereof.

### Useful substance

Useful substance refers to a vast array of biochemical compounds including, but not limited to, the following. Useful substances are either commercial products (e.g., pharmaceuticals, proteins including pharmaceutical biologics such as immunotoxins, antibodies, or interferons, or industrial enzymes (such as amylase, glucanase, pullulanase, invertase), peptides (including peptide hormones and peptide hormone analogs), vitamins including beta-carotene, riboflavin, or vitamin B12), amino acids (e.g., glutamic acid, lysine, tryptophane), or biochemical buildings blocks (e.g., 1,4 diacids: succinic acid, fumaric acid, and acetic acid, organic acids, 2,5-furan dicarboxylic acid, 3-hydroxy propionic acid, aspartic acid, glucaric acid, glutamic acid, itaconic acid, levulinic acid, 3-hydroxyburyrolactone, glycerol, sorbitol, xylitol, arabinitol, lactic acid, citric acid, gluconic acid), or commodity chemicals (e.g., ethanol, butanol, acetone).

### Fermentation medium

Fermentation medium refers to the liquid medium in which the fermentation is performed or in which the fermenting organism is grown. This fermentation medium includes a suitable source of carbon, such as sugars, starch, cellulose, hemicellulose, as well as any growth stimulators necessary for the growth or metabolic activation of the fermenting organism. The fermentation medium can refer to the medium either before or after the fermenting organism has been inoculated, has grown, or in which it has been incubated. The fermentation medium may comprise enzymes such as amylase (alpha or beta-amylase, or gluco-amylase or maltogenic amylase) or cellulases, or may result from the effect of such enzymes.

### Fermenting organism

The fermenting organism refers to any organism, including bacteria, yeast, fungi, or mammalian or insect cells that are used in the invention to produce useful substances. For the avoidance of the doubt, fermenting organisms include wild type organisms and organisms that have been genetically modified such as to confer to them suitable properties for the production of useful substances or for the utilization of various fermentation media. Organisms for producing ethanol include, but are not limited to: *Saccharomyces* and typically *Saccharomyces cerevisiae, Schizosaccaromyces* and typically *Schizosaccaromyces pombe, Aspergillus* and typically *Aspergillus niger and Aspergillus oryzae, Penicillium* and typically *Penicillium roquefortii, Kluyveromyces, Corynebacterium* and typically *Corynebacterium glutamicum* and *Corynebacterium efficiens, Bacillus* and typically *Bacillus subtilis* and *Bacillus megaterium, Lactobacillus* and typically *Lactobacillus lactis, Escherichia* and typically *Escherichia coli, Zymomonas* and typically *Zymomonas mobilis,* algae and typically Cyanobacteria, mammalian cells and typically Chinese Hamster Ovary cells and plants cells. Specifically, guidance on using or engineering *C. glutamicum* can be found elsewhere (Vertès et al., 2005b).

### Cell culture

A cell culture is a batch of cells that is obtained by inoculating an appropriate fermentation medium, and growing said cells under appropriate conditions of temperature, pressure, and oxygen tension. A cell culture can be also incubated under conditions of repressed cell division, in batch, fed-batch, continuous, or semi-continuous mode, and can be grown either in batch, fed-batch, continuous, or semi-continuous mode. The cell concentration of a cell culture can be modified by collecting cells by centrifugation or filtration and resuspending said cells in an appropriate volume of fermentation medium.

### Source of carbon

Source of carbon refers to any carbon-containing biochemical compound that can be used by microorganisms to grow. Sources of carbon particularly include sugars, such as glucose, fructose, xylose, arabinose, or saccharose, starch, cellulose and hemicellulose - also referred elsewhere in the scientific literature as lignocellulosic materials. Sugars, starch, and lignocellulosic materials typically derive, eventually by means of a pre-treatment step or a simultaneous saccharification step, from plant materials including, but not limited to, cereals, maize, wheat, barley, rice, whole grains, tubers, roots, molasses, fruits and fruit residues, sugar cane, sugar beet, potatoes, soft wood and hard wood. Sugars, starch, and lignocellulosic materials may also derived from municipal solid wastes, recycled paper and carton, or industrial waste or recycled wood such as recycled construction wood. Example of saccharification steps can be found in the production processes currently used to produce ethanol using *Saccharomyces cerevisiae* (Lin and Tanaka, 2006).

### Growth stimulator

In an embodiment of the invention, growth stimulator can be added or removed from the fermentation medium such as to promote or halt growth. Typical growth stimulators include, but are not limited to, minerals and vitamins, and particularly biotin, panthotenic acid, nicotinic acid, thiamine, para-aminobenzoic acid, folic acid, riboflavin, and vitamin A, B, C, D, and E

### Bacteriostatic compound

In an embodiment of the invention, bacteriostatic compounds can be added to halt cellular growth or dramatically decrease the rate of cellular growth. Bacteriostatic compounds can also be generated by the fermenting organism, typically a recombinant organism, upon addition of a suitable bacteriostasis inducer, with a bacteriostasis inducer being a chemical or biochemical compound that triggers expression of a gene and production of the corresponding gene product, said gene product is a bacteriostatic compound.

### Cellular compartment

Cellular compartment refers to extracellular, intracellular, membrane, periplasm, organelle compartments that constitute a cell and its surrounding medium. More than one cellular compartment can refer to any combination thereof, including but not limited to the sequential use of the extracellular compartment in different fermentation that make use of the same cell culture.

### Downstream processing

Downstream processing techniques referred to in this invention are any method known in the art to purify useful substances. Particularly, the spent fermentation medium may be distilled to recover the fermentation product (i.e., the useful substance), such as ethanol. Ethanol thus produced can particularly be used as fuel ethanol or industrial ethanol. Likewise, amino acids from *C. glutamicum* fermentation or proteins from *E. coli* fermentation can be recovered using any techniques known in the art.

### REFERENCES

Aristidou A., and M. Pentilla. 2000. Metabolic engineering applications to renewable resource utilization. Curr. Opin. Biotechnol. 11:187-198
**Avron M., and A. Ben-Amotz, 1978. Productin of glycerol, carotenes, and algae meal.** US Patent 4,199,895
Barreiro C., E. González-Lavado, M. Pátek, and J.F. Martin. 2004. Transcriptional análisis of the groES-groEL1, groEl2, and dnaK genes in Corynebacterium glutamicum: characterization of heat shock-induced promoters. Appl. Env. Microbiol. 186:4813-4817
Bekers M., D. Upite, E. Kaminska, M. Grube, J. Laukevics, I. Vina, A. Vigants, anad P. Zirkmanis. 2003. Fructan synthesis by intra- and extracellular Zymomonas mobilis levansucrase after simultaneous production of ethanol and levan. Acta Biotechnol. 23:85-93
Bellissimi E., and W.M. Ingledew. 2005. Metabolic acclimatization: preparing active dry yeast for fuel ethanol production. Process Biochem. 40:2205-2213
Bothast R.J., N.N. Nichols, and B.S. Dien. 1999. Fermentations with new recombinant organisms. Biotechnol. Prog. 15:867-875
Clements L.D., and D.L. van Dyne. 2006. in Biorefineries - industrial processes and products, vol. 1 (eds. B. Kamm, P.R. Grüber & M. Kamm), pp. 115-127, Weinheim, Germany: Wiley
**Crafton C.M., and P.J. Rayapati, 2001.** Nucleotide sequences for transcriptional regulation in *Corynebacterium glutamicum.* United States Patent Application 2003/0017553 A1
Dien B.S., M.A. Cotta, and T.W. Jeffries. 2003. Bacteria engineered for fuel ethanol production: current status. Appl. Microbiol. Biotechnol. 63:258-266
Doelle H.W., L. Kirk, R. Crittenden, H. Toh, and M.B. Doelle. 1993. Zymomonas mobilis-science and industrial application. Crit. Rev. Biotechnol. 13:57-98
de Carvalho Lima K.G., C.M. Takahashi, and F. Alterthum. 2002. Ethanol production from corn cob hydrolyzates by Escherichia coli KO11. J. Ind. Microbiol. Biotechnol. 29:124-128
Gulati M. et al. 1996. Assessment of ethanol production options from corn products. Bioresourc. Technol. 58:253-264
**Hindcliffe E., and E. Kenny. 1986. Process for the isolation of fermentation products.** European Patent 0201239 A1
**Hori K., and H. Unho. 2003. Simultaneous biosynthesis of polyhydroxy alkanoic acid and rhamnolipid involves cultivating *Pseudomonas* in culture medium, centrifuging, extracting acid from microbial cells and extracting rhamnolipid from supernatant.** Japanese Patent 2003 052368 A
Ingram L.O. et al. 1999. Enteric bacterial catalysts for fuel ethanol production. Bitoechnol. Prog. 15:855-866
Inui M., H. Kawaguchi, S. Murakami, A.A. Vertès, and H. Yukawa. 2004a. Metabolic engineering of Corynebacterium glutamicum for fuel ethanol production under oxygen-deprivation conditions. J. mol. Microbiol. Biotechnol. 8:243-254
Inui M., et al. 2004b. Metabolic analysis of Corynebacterium glutamicum during lactate and succinate productions under oxygen deprivation conditions. J. Mol. Microbiol. Biotechnol. 7:182-196
Karlscheuer R., T. Stölting, and A. Steinbüchel. 2006. Microdiesel: Escherichia coli engineered for fuel production. Microbiology 152:2529-2536
**Kataoka H., M. Matsumura, H. Miyabe, and K. Miyamoto. 1990. Manufacturing squalene and ethanol comprises culturing squalene generating bacterium belonging to *Saccharomyces* anaerobically.** Japanese Patent 4179487
Keim C.R. 1983. Technology and economics of fermentation alcohol - an update. Enz. Microb. Technol. 5:103-114
Klinke H.B., A.B. Thomsen, and B.K. Ahring. 2004. Inhibition of ethanol-producing yeast and bacteria by degradation products produced during pre-treatment of biomass. App;. Microbiol. Biotechnol. 66:10-26
**Kreutzer C., S. Hans, M. Rieping, B. Mockel, W. Pfefferle, L. Eggeling, H. Sahm, and M. Patek. 2005.** L-lysine-producing Corynebacteria and process for the preparation of L-lysine. US Patent Application 20050074791
Lin Y., and S. Tanaka. 2006. Ethanol fermentation from biomass resources: current state and prospects. Appl. Microbiol. Biotechnol. 69:627-642
Nichols N.N., B.S. Dien, and R.J. Bothast. 2003. Engineering lactic acid bacteria with pyruvate decarboylase and alcohol dehydrogenase genes for ethanol production from Zymomonas mobilis. J. Ind. Microb. Biotechnol. 30:315-321
Nigam J.N. 2000. Continuous ethanol production from pineapple cannery waste using immobilized yeast cells. J. Biotechnol. 80:189-193
O'Brien D.J., G.E. Senske, M.J. Kurantz, and J.C. Craig. 2004. Ethanol recovery from corn fiber hydrolyzate fermentations by pervaporation. Bioresourc. Technol. 92:15-19
**Ohara H., and M. Yahata. 1996.** Method for producing L-lactic acid with high optical purity using bacillus strains. European Patent 0 770 684 A2
Park I.S., J.H. Kim, and B.G. Kim. 2005. The effect of ftsZ mutation on the production of recombinant protein in Bacillus subtilis. Appl. Microbiol. Biotechnol. 69:57-64
Peters D. 2006. Carbohydrates for fermentation. Biotechnol. J. 1:806-814
Peters-Wendisch P., M. Stolz, H. Etterich, N. Kennerknecht, H. Sahm, and L. Eggeling. 2005. Metabolic engineering of Corynebacterium glutamicum for L-serine production. Appl. Env. Microbiol. 71:7139-7144
Rosenfeld E., B. Beauvoit, B. Blondin, and J.M. Salmon. 2003. Oxygen consumption by anaerobic Saccharomyces cerevisiae under enological conditions: effect on fermentation kinetics. Appl. Environ. Microbiol. 69:13-121
**Scharif-Afschaar A., and A. Quesada-Chanto. Continuous fermentation process which is useful for the simultaneous optimal production of propionic acid and vitamin B12.** European Patent 0 668 359 A1
Simic P., H. Sahm, and L. Eggeling. 2001. L-threonine export: use of peptides to identify a new translocator from Corynebacterium glutamicum. J. Bacteriol. 183:5317-5324
Soetaert W. and E. Vandamme. 2006. The impact of industrial biotechnology. Biotechnol. J. 1:756-769
Talarico L.A., M.A. gil, L.P. Yomano, L.O. Ingram, and J.A. Maupin-Furlow. 2005. Construction and expression of an ethanol production operon in Gram-positive bacteria. Microbiology. 151:4023-4031
Taylor F., M.J. Kurantz, N. Goldberg, A.J. McAloon, and J.C. Craig. 2000. Dry grind process for fuel ethanol by continuous fermentation and stripping. Biotechnol. Prog. 16:541-547
Thomsen M.H. 2005. Complex media from processing of agricultural crops for microbial fermentation. Appl. Microbiol. Biotechnol. 68:598-606
Vertès A.A., M. Inui, and H. Yukawa. 2005. In 14th European Biomass Conference & Exhibition. Biomass for energy, industry, and climate protection, pp 1068-1071, Paris
Vertès A.A., M. Inui, and H. Yukawa. 2005b. Manipulating Corynebacteria, from genes to chromosomes. Appl. Environ. Microbiol. 71:7633-7642
Vertès A.A., M. Inui, and H. Yukawa. 2007. Alternative technologies for biotechnological fuel ethanol manufacturing. J. Chem. Technol. Biotechnol. 82:693-697
Webb C., W.R. Koutinas, and R. Wang. 2004. Developing a sustainable bioprocessing strategy based on a generic feedstock. Adv. Biochem. Eng. Biotechnol. 87:195-268
Wennerhold J., and M. Bott. 2006. The DtxR regulon of Corynebacterium glutamicum. J. Bacteriol. 188:2907-2918
**Wolf A., N. Schischka, T. Hermann, S. Morbach, and R. Kraemer. 2005.** Process fort the fermentative preparation of L-amino acids using coryneform bacteria. US Patent Application 20050266536
Wood B.E., D.S. Beall, and L.O. Ingram. 1997. Production of recombinant bacterial endoglucanase as a coproduct with ethanol during fermentation using derivatives of Escherichia coli KO11. Biotechnol. Bioeng.
Yukawa H., C.A. Omumasaba, H. Nonaka, P. Kós, N. Okai, N. Suzuki, M. Suda, Y. Tsuge, J. Watanabe, Y. Ikeda, A.A. Vertès, and M. Inui. 2007. Comparative analysis of the Corynebacterium glutamicum group and complete genome sequence of strain R. Microbiol. 153:1042-1058
Zhou S., L.P. Yomano, A.Z. Saleh, F.C. Davis, H.C. Aldrich, and L.O. Ingram. 1999. Enhancement of expression and apparent secretion of Erwinia chrysanthemi endoglucanase (encoded by celZ) in Escherichia coli B. Appl. Env. Microbiol. 65:2439-2445
Zupancic T.J., J.D. Kittle, B.D. Baker, C.J. Miller, D.T. Palmer, Y. Asai, M. Inui, A. Vertès, M. Kobayashi, Y. Kurusu, and H. Yukawa. 1995. Isolation of promoters from Brevibacterium flavum strain MJ233C and comparison of their gene expression levels in B. flavum and Escherichia coli. FEMS Microbiol. Letters 131:121-126

## Claims

1. A process of fermenting sources of carbon to produce more than one useful substance using more than one cellular compartment (extracellular, intracellular, membrane, periplasm, organelles) either sequentially or in parallel using the same cell culture.

2. The process of claim 1 wherein one of the compartments is the extracellular compartment and another of the compartments is the intracellular or periplasmic compartment or an organelle.

3. The process of claim 1 wherein one of the compartments is the extracellular compartment and another of the compartments is a different extracellular compartment obtained by collecting cells by centrifugation or filtration and resuspending said cells in fermentation medium.

4. The process of any of claims 1-3 wherein cells are incubated at high cell densities, typically higher than 5 times the cell density attained at the end of the logarithmic phase when growing fermenting organism in fermentation medium.

5. The process of any of claims 1-4 where more than two compartments are used.

6. The process of any of claims 1-5 wherein the fermentation is performed in batch or fed-batch mode or wherein the fermentation is performed in continuous or semi-continuous mode

7. The process of any of claims 1-5 wherein the fermentation is performed using a combination of batch or fed-batch mode and a continuous or semi-continuous mode

8. The process of any of claims 1-7 wherein conditions of repression of cell replication are used at one of the steps of the multiplex fermentation process. Said conditions of repression of cell replication are achieved by either adding a bacteriostatic chemical or biological compound or inducing production of such by the fermenting organism, or varying a physical growth parameter such as temperature, oxygen tension, or cellular concentration, or by removing or sequestering from the fermentation medium a growth stimulator.

9. The process of any of claims 1-8 wherein one of the useful substances produced is ethanol or an organic acid, and wherein another of the useful substances produced is an amino acid, a protein, a peptide, a hormone, a hormone analog, an enzyme, a nucleic acid, a vitamin, an antibody, an interferon, or an immunotoxin.

10. The process of any of claims 1-9 wherein the fermentation organism is a Gram-positive organism, such as a *Bacillus, a Lactobacillus, a Corynebacterium,* or a *Streptomyces* and their recombinant derivatives, or a yeast and particularly *Saccharomyces cerevisiae* and recombinant derivatives, or a filamentous fungus and particularly *Aspergillus niger* and *Aspergillus oryzae* and their recombinant derivatives, or a Gram-negative organism and particularly *Escherichia coli* or *Zymomonas mobilis* and their recombinant derivatives, or a *Clostridium* and particularly *Clostridium acetobutylicum* and recombinant derivatives.

11. The process of any of claims 1-10 wherein the fermentation organism is *Corynebacterium glutamicum* and recombinant derivatives.

12. The process of any of claims 1-10 wherein fermentation organism is *Escherichia coli* and wherein proteins are produced as inclusion bodies in the periplasmic space.

13. The process of any of claims 1-12 wherein the membrane compartment is utilized to produce one or more enzyme.

14. The process of any of claims 1-13 wherein one of the useful substances is recovered by distillation.

## Amended claims

### Amended claims in accordance with Rule 137(2) EPC.

**1.** A process of fermenting sources of carbon to produce more than one useful substance using more than one cellular compartment (extracellular, intracellular, membrane, periplasm, organelles) either sequentially or in parallel using the same cell culture, one of these substances being an amino acid, a vitamin or a peptide (Group 1 substance) and another of these substances being ethanol or an organic acid or any substance recovered by distillation (Group 2 substance), and wherein a Group 1 substance is produced intracellularly and a Group 2 substance is produced extracellularly.

**2.** The process of claim 1 wherein one gene or more of the production organism that are involved in the export of the amino acid, vitamin or peptide (Group 1 substance) are inactivated by any means, such as but not limited to gene deletion, gene disruption and replacement, or gene silencing.

**3.** A process of fermenting sources of carbon to produce more than one useful substance using the same cell culture, one of these substances being an amino acid, a vitamin or a peptide (Group 1 substance) and another of these substances being ethanol or an organic acid or any substance recovered by distillation (Group 2 substance), and wherein both Group 1 substance and Group 2 substance are produced extracellularly but in sequence.

**4.** The process of claim 3 wherein an amino acid, a vitamin or a peptide is produced extracellularly in the first stage of the fermentation and ethanol or an organic acid or any substance that can be recovered by distillation is produced extracellularly in a second stage of the fermentation, with the switch between fermentation phases being controlled by a variation in temperature or the concentration of a particular element, for example -but not limited to- iron.

**5.** The process of claim 4 wherein the second phase of the fermentation is performed at very high cellular concentrations, typically 5 to 50 times the cellular concentration typically attained at the end of the logarithmic phase of growth

**6.** The process of any of claims 1-5, wherein lysine and ethanol or an organic acid are produced

**7.** The process of any of claims 1-5 wherein serine and ethanol or an organic acid are produced

**8.** The process of any of claims 1-7 wherein more than two compartments are used.

**9.** The process of any of claims 1-8 wherein the fermentation is performed in batch or fed-batch mode or wherein the fermentation is performed in continuous or semi-continuous mode

**10.** The process of any of claims 1-8 wherein the fermentation is performed using a combination of batch or fed-batch mode and a continuous or semi-continuous mode

**11.** The process of any of claims 1-8 wherein conditions of repression of cell replication are used at one of the steps of the multiplex fermentation process. Said conditions of repression of cell replication are achieved by either adding a bacteriostatic chemical or biological compound or inducing production of such by the fermenting organism, or varying a physical growth parameter such as temperature, oxygen tension, or cellular concentration, or by removing or sequestering from the fermentation medium a growth stimulator such as biotin.

**12.** The process of any of claims 1-11 wherein the fermentation organism is a Gram-positive organism, such as a *Bacillus*, a *Lactobacillus*, a *Corynebacterium,* or a *Streptomyces* and their recombinant derivatives, or a yeast and particularly *Saccharomyces cerevisiae* and recombinant derivatives, or a filamentous fungus and particularly *Aspergillus niger* and *Aspergillus oryzae* and their recombinant derivatives, or a Gram-negative organism and particularly *Escherichia coli* or *Zymomonas mobilis* and their recombinant derivatives, or a *Clostridium* and particularly *Clostridium acetobutylicum* and recombinant derivatives.

**13.** The process of any of claims 1-11 wherein the fermentation organism is *Corynebacterium glutamicum* and recombinant derivatives.

**14.** pAV740 and pAV741 constructs
